Europäisches Patentamt

European Patent Office

Office européen des brevets

(19)

(11) Veröffentlichungsnummer: **0 340 604 B1**

(12) **EUROPÄISCHE PATENTSCHRIFT**

(45) Veröffentlichungstag der Patentschrift: **02.03.94**

(51) Int. Cl.⁵: **C12P 21/02**, C12P 21/08, A61K 39/395

(21) Anmeldenummer: **89107438.7**

(22) Anmeldetag: **25.04.89**

(54) **Monoklonaler Antikörper und seine Verwendung.**

(30) Priorität: **06.05.88 DE 3815472**

(43) Veröffentlichungstag der Anmeldung:
**08.11.89 Patentblatt 89/45**

(45) Bekanntmachung des Hinweises auf die
Patenterteilung:
**02.03.94 Patentblatt 94/09**

(84) Benannte Vertragsstaaten:
**AT BE CH DE ES FR GB GR IT LI LU NL SE**

(56) Entgegenhaltungen:
**EP-A- 0 241 811**

**The Lancet, 13 Juni 1987, Seiten 1339-1342;
J.P. Soulillou et al.: "Prevention of rejection
of kidney transplants by monoclonal antibody directed against interleukin"**

**Chemical Abstracts, Band 103, Nr. 9, 2 September 1985, Seite 499, Zusammenfassung
nr 69519p, Columbus, Ohio, US; L.A. Rubin et
al.: "A monoclonal antibody 7G7/B6, binds to
an epitope on the human interleukin-2 (IL-2)
receptor that is distinct from that recognized by IL-2 or anti-Tac"**

(73) Patentinhaber: **Innothérapie S.A.
1 Bld Alexandre Fleming
F-25020 Besançon(FR)**

(72) Erfinder: **Wijdenes, John, Prof.
Rue du Coteau
Auxon-Dessus
F-25870 Geneuille(FR)**
Erfinder: **Herve, Patrick, Prof.
Chemin des Terre Rouges
F-25770 Franois(FR)**
Erfinder: **Clement, Claude
Essertenne
F-70100 Gray(FR)**
Erfinder: **Morel-Fourrier, Brigitte
2 Rue Leonard de Vinci
F-25000 Besançon(FR)**
Erfinder: **Peters, André, Prof.
2 Place Jean Cornet
F-25000 Besançon(FR)**

The Journal of Immunology, Band 139, Nr. 5, 1 September 1987, Seiten1550-1556, The American Association of Immunologists, US; LE THI BICH-THUY et al.: "Direct activation of human resting T cells by IL 2: The role of an IL 2 receptor distinct from the Tac protein"

Progress in Leukocyte Biology (US) 1988(1989), Band 0, Nr. 0; Cellular Basis of Immune Modulation, Seiten 551-555 (19th International Leukocyte Culture Conference, Banff, Alberta, Canada, Mai 8-12, 1988) J. Wijdenes et al.: "A monoclonal antibody against the human IL-2 receptor with high inhibitory activity on IL-2 induced proliferation of T cells, experimental and clinical results"

74 Vertreter: **Wolff, Hans Joachim, Dr.jur. Dipl.-Chem. et al BEIL, WOLFF & BEIL, Rechtsanwälte, Postfach 80 01 40 D-65901 Frankfurt am Main (DE)**

EP 0 340 604 B1

**Beschreibung**

Die Erfindung betrifft eine neue Hybridoma-Zellinie und einen davon abgeleiteten monoklonalen Antikörper, der ein Antigen auf aktivierten T-Zellen, den IL-2-Rezeptor erkennt und die Interleukin-2-(IL-2)-abhängige Proliferation inhibiert.

Die Erfindung betrifft weiterhin die Verwendung dieses monoklonalen Antikörpers für therapeutische und auch diagnostische Zwecke.

Interleukin-2 (IL-2), ursprünglich als T-Zell-Wachstumsfaktor (TCGF) (L.A. Aarden, J.Immunol. 123, 2928, 1979) bezeichnet, ist einer der wesentlichen Mediatoren der zellulären Immumantwort.

Die durch IL-2 vermittelte Immunantwort resultiert dabei aus seiner Wechselwirkung mit einem hochaffinen Zelloberflächenrezeptor. Dieser Interleukin-2-Rezeptor (IL-2 R) besteht aus zwei nicht kovalent gebundenen Untereinheiten: einem kleinen Teil (Mr = 55 KDa, Tac-Antigen) und einem schweren Teil (Mr = 75 KDa). Dabei wurde gezeigt (K.A. Smith, Immunology Today, Vol. 9, 1988, 36), dass sowohl der leichte als auch der schwere Teil eine Bindungsaffinität zum IL-2 aufweisen ($K_D$ = $10^{-8}$ M für den leichten und $10^{-9}$ M für den schweren Teil). Beide Untereinheiten bilden den hochaffinen IL-2-Rezeptor mit einer Bindungskonstanten von $10^{-11}$ M.

Zur Inhibierung der Bindung des IL-2 an seinen Rezeptor werden daher spezifische Substanzen benötigt, die diese Affinität des IL-2 zum Rezeptor z.B. durch Interaktion mit der kleineren Untereinheit und/oder mit der grösseren Kette unterbinden.

Als hierfür geeignet erwiesen sich monoklonale Antikörper (MAK), die nach bekannten Methoden (C. Milstein, G.Köhler, Nature 256 (1975), 495) bereitgestellt werden können. Die auf diese Art gewonnenen MAK's erkennen bestimmte Epitope des IL-2-R-Moleküls. Bisher sind auf der leichten Kette 3 Epitope bekannt (T. Diamantstein, Behring Inst. Mitt. 81,73 (1987)).

Uchiyama et al(J. Immunol.126, 1398 (1981) hat den ersten MAK beschrieben, der das Tac-Antigen (Epitop), d.h. die kleine Kette des IL-2 R erkennt.

Die EP-A 0 241 811 beschreibt zwei monoklonale Antikörper, welche ebenfalls nach der obengenannten Methode erhalten werden. Diese reagieren spezifisch mit aktivierten T- und B-Zellen, sind aber inaktiv gegenüber ruhenden Lymphozyten. Einer der beiden MAK's, ein Anti-Tac-Analoges, tritt hierbei mit dem Tac-Antigen, also der kleineren Kette des Rezeptormoleküls, in Wechselwirkung. Der zweite MAK tritt offenbar ebenfalls mit der kleinen Untereinheit des Rezeptors in Wechselwirkung. Er erkennt jedoch eine vom Tac-Epitop verschiedene Determinante. Er inhibiert sowohl die IL-2-abhängige Proliferation als auch die Bindung des IL-2 an den IL-2 R. Die Effektivität dieser MAK's wurde in klinischen Versuchen jedoch nicht nachgewiesen.

Die EP-A 0 240 344 betrifft Anti-CD 4 MAK's und Anti-Tac-Analoge (CD 25). Ihre Wirkungsweise bezüglich der Immunsuppression und zwar der Verhinderung der Transplantatabstossung wurde an Mäusen und Ratten getestet.

Hierbei ist jedoch eine Übertragung der Ergebnisse auf IL-2-abhängige Reaktionen beim Menschen nicht möglich, da die genannten MAK's Spezies spezifisch sind und nur gegen den Maus-IL-2 R wirksam sind, d.h. eine klinische Wirksamkeit wurde nicht erbracht.

J.P. Soulillou et al (The Lancet, 13.Juni (1987), 1339) beschreiben die Aktivität eines MAK's gegen den humanen IL-2 R bei der Bekämpfung der Nierentransplantatabstossung am Menschen. Die Art und Weise der Interaktion dieses als 33B31 bezeichneten MAK's ist nicht bekannt. Hierbei sind relativ hohe Dosierungen erforderlich. Häufig treten dabei - aufgrund der Menge an Fremdprotein - signifikante Nebenwirkungen (Fieber, Unverträglichkeit und Antikörperbildung) auf.

Der Erfindung liegt daher die Aufgabe zugrunde, einen monoklonalen Antikörper bereitzustellen, welcher geeignet ist, alle IL-2 abhängigen Schritte der zellulären Immunabwehr am Menschen zu hemmen, aufzuhalten oder zu unterdrücken, um somit eine dauerhafte Toleranz des als fremd erkannten Gewebes zu erreichen. Dabei soll der MAK in so geringen Mengen sowohl prophylaktisch als auch therapeutisch wirksam sein, dass keine oder nur sehr schwache Nebenwirkungen während oder als Folge der Therapie auftreten.

Diese Aufgabe wird erfindungsgemäss gelöst, indem nach einer an sich bekannten Methode von C.Milstein/G. Köhler eine Hybridoma-Zellinie bereitgestellt wird, die einen murinen monoklonalen AK der Klasse $IgG_1$ gegen den humanen IL-2-R produziert, welcher den humanen IL-2-Rezeptor erkennt und mit der 55 KDa-Untereinheit des IL-2-Rezeptors spezifisch wechselwirkt, die Bindung von humanem IL-2 an seinen Rezeptor inhibiert und/oder verdrängt und eine bereits ablaufende allogene Lymphozyten-Reaktion inhibiert.

Aus dieser Zellinie der C.N.C.M-Hinterlegungsnummer I-752 können weiterhin Class-switch-Varianten der murinen Immunglobuline, wie z.B. $IgG_{2a}$ und $IgG_{2b}$, $IgG_3$ und IgM und andere Immunglobulinklassen,

3

isoliert werden, die die gleichen Eigenschaften besitzen.

Der erfindungsgemässe MAK, im folgenden als B.B. 10 bezeichnet, kompetiert die Bindung von IL-2 an den IL-2 R auf humanen T-Zellen und inhibiert die IL-2-induzierte Proliferation von aktivierten T-Zellen.

Darüberhinaus erfolgt eine Inhibierung der humanen gemischten Lymphozytenreaktion. Dabei hat sich gezeigt, dass der MAK an ein von Tac verschiedenes Epitop des humanen Il-2-Rezeptors bindet. Insgesamt ist das Protein daher geeignet zur Therapie und Prophylaxe von Erkrankungen, wie z.B. Hyperimmun-Syndrome, Graft versus Host Krankheit, Host versus Graft Krankheit, bei Transplantationen des Knochenmarks, von Nieren, Herz, Lunge, Pankreas, Haut, Leber u.a.) oder T-Zell-abhängigen allergischen und autoimmunen Erkrankungen (Myocarditis, Diabetis, Myasthenia gravis, Lupus Erythematodes, Morbus Crohn, Multiple Sklerose, AIDS, Enzephalomyelitis, Arthritis u.a. mehr) und von IL-2 Rezeptor exprimierenden Tumor-Erkrankungen, wie z.B. T-Zell-Leukämie.

Hierzu kann der MAK sowohl als solcher als auch gekoppelt, z.B. mit magnetischen Beads, radioaktiven Substanzen oder Arzneimitteln oder in Liposomen eingekapselt, eingesetzt werden.

Darüber hinaus ist der MAK B.B. 10 geeignet als diagnostisches Reagenz, um den humanen IL-2-Rezeptor auf Zelloberflächen oder in Körperflüssigkeiten nachzuweisen. Dadurch ist es mit Hilfe des erfindungsgemässen MAK's möglich, Zellen, welche IL-2 R exprimieren, zu identifizieren. Bei einer derartigen Anwendung wird der MAK vorzugsweise an fluoreszierende oder andere farbbildende Substanzen oder an eine radioaktive Substanz gekoppelt, oder man arbeitet mit einem zweiten markierten Antikörper, der spezifisch gegen Maus-Immunglobulin gerichtet ist.

Darüberhinaus kann man in Kombination mit einem zweiten anti IL-2 R-Antikörper einen ELISA und/oder RIA zur Messung von freiem, abgelöstem IL-2 R in Körperflüssigkeiten entwickeln.

Weiterhin ist der erfindungsgemässe MAK geeignet zur Herstellung von Chimären, deren konstanter Teil humanen Ursprungs (humanes Ig) und deren variabler, insbesondere hypervariabler Teil, murinen Ursprungs ist. Die Chimären können gleichfalls als solche oder gekoppelt mit magnetischen Beads, radioaktiven Substanzen, zusammen mit anderen Arzneimitteln oder eingekapselt in Liposomen gleichermassen zur Therapie oder Prophylaxe IL-2-abhängiger Erkrankungen beim Menschen eingesetzt werden.

Die Erfindung wird anhand der folgenden Beispiele näher erläutert.

## I. Herstellung des MAK

### Beispiel 1: Herstellung des Antigens (IL-2 R)

T-Zellen aus humanen peripheren Blutlymphozyten (PBL) wurden mittels Schafserythrozyten-Rosettierung isoliert und 4 Tage in einem Kulturmedium (RPMI 1640, 10% Fötalkälberserum (FCS) der Firma Flow, lot 126075, und 10 $\mu$g/ml Phytohämaglutenin (PHA) im Brutschrank (5% $CO_2$, 95% Luftfeuchte) bei 37°C inkubiert.

Die auf diese Weise kultivierten Zellen dienten im folgenden als Quelle für den humanen IL-2 R, hier als PHA/PBL bezeichnet.

### Beispiel 2: Immunisierung, Fusion, Klonierung und Gewinnung des monoklonalen Antikörpers B.B. 10

Weibliche BALB/c Mäuse wurden intraperitoneal 3 mal in zweiwöchigem Abstand mit 5 x $10^6$ PHA/PBL's immunisiert. Die dritte Immunisierung wurde intravenös verabreicht und die Milzzellen 4 Tage später entnommen und fusioniert. Die Fusion wurde wie folgt durchgeführt:

Die immunisierten Milzzellen wurden mit AG 8653 Maus-Myeloma-Zellen im Verhältnis 5:1 in Gegenwart von Polyethylenglykol fusioniert (Kearney et al (1978), J. Immunol. 123, 1548).

Die fusionierte Zellsuspension wurde einmal gewaschen und im Selektionsmedium (RPMI 1640, 10% hitzeinaktiviertes Pferdeserum), 4 mM Glutamin, HAT: Hypoxanthin 13,6 mg/l; Aminopterin 0,17 mg/l und 10 $\mu$g/ml Insulin) kultiviert. Zehn Tage nach der Fusion wurden Überstände von Kulturen, die Hybridomawachstum zeigten, auf anti-IL-2 R MAK-Produktion getestet. Dazu wurden PHA/PBL's und PBL's (3 x $10^5$ in 50 $\mu$l 1% Rinderserumalbumin/PBS) mit 10 $\mu$l Testüberstand oder Kontroll-MAK inkubiert. Als Kontroll-MAK's wurden in diesem und den nachfolgenden Beispielen die anti-Tac-analogen MAK's BF 2 und BG 8, eingesetzt (CRTS, Besancon). Der gebundene MAK wurde mittels eines FITC-markierten anti-Maus-Antikörpers im Cytofluorometer (Ortho 50H - Ortho) nachgewiesen. Von 1187 getesteten Überständen zeigten 8 Überstände eine signifikante Bindung an aktivierten T-Zellen, ohne an ruhende T-Zellen zu binden.

Nach 4 Klonierungsschritten unter Benutzung der "limiting dilution" Methode (Aussaatdichte 0,2 Zellen/Kultur) wurde der B.B.10 Klon isoliert.

B.B.10 ist ein Maus-IgG$_1$-MAK mit einer Kappa leichten Kette und weist eine signifikante Bindung an aktivierten T-Zellen auf.

Beispiel 3: In vivo-Produktion und Reinigung des MAK B.B.10

Der anti-IL-2-R MAK B.B.10 wurde in grossen Mengen in vivo durch intraperitoneale Injektion von B.B.10-Hybridomazellen in BALB/c Mäusen produziert. Eine Woche vor der Hybridomazellinjektion wurden die Mäuse mit 1,0 ml Pristan intraperitoneal geprimt. 8 - 14 Tage nach der Hybridomazellinjektion konnte Ascitesflüssigkeit abgenommen werden.

Der MAK wurde anschliessend durch Ammoniumsulfatfällung (45% Sättigung) aus der Ascitesflüssigkeit präzipitiert, auf 0,02 mM Tris, pH = 7,7, umgepuffert und an eine Q-Sepharose-Säule gebunden. Auf dieser Säule wurde der MAK mit 1% Tween 20 in 0,02 mM Tris, pH = 7,7, gewaschen und anschliessend mit 0,35 M NaCl (pH = 7,7) von der Säule eluiert.

Für therapeutische Zwecke wurde der MAK auf physiologischen PBS-Puffer (phosphatgepufferte Saline) umgepuffert.

II. Biologische Aktivität des MAK

Beispiel 4a: Inhibierung der IL-2-induzierten Proliferation aktivierter T-Zellen durch MAK B.B.10

PHA-aktivierte T-Zellen (5 x $10^4$/Kultur) wurden 24 h in RPMI 1640, 10% FCS kultiviert. Dem Medium wurden zu Beginn die in den Tabellen 1 und 2 angegebenen Mengen IL-2 (von Biotest, Lymphocult-T$^{(R)}$), MAK B.B.10 bzw. die Kontroll-MAK's BF 2 und BG 8 und H$^3$-Thymidin zugesetzt. Die DNA-Synthese wird als H$^3$-Thymidin-Einbau bestimmt. Hierzu wird durch Messung der Radioaktivität mittels Standard-Flüssig-Szintillationstechniken das Zellwachstum bzw. die Inhibierung ermittelt.

Die Ergebnisse sind in den Tabellen 1 und 2 zusammengefasst:

Tabelle 1

| IL-2 U/Napf | MAK µg/Napf | Gemessene Radioaktivität cpm ± SD | | |
|---|---|---|---|---|
| | | B.B.10 | BF 2 | BG8 |
| 0 | 0 | 570± 152 | nb | nb |
| 1 | 0 | 3554 ± 140 | | |
| | $10^{-2}$ | 2117 ± 142 | 2734 ± 143 | 3789 ± 147 |
| | $10^{-1}$ | 1430 ± 147 | 2401 ± 145 | 1530 ± 159 |
| | 1 | 913 ± 150 | 1595 ± 148 | 1077 ± 158 |
| | 10 | 943 ± 151 | 1099 ± 144 | 1009 ± 163 |
| 10 | 0 | 5250 ± 140 | | |
| | $10^{-2}$ | 4786 ± 139 | 6475 ± 143 | 6413 ± 145 |
| | $10^{-1}$ | 3907 ± 142 | 4879 ± 141 | 6759 ± 148 |
| | 1 | 3220 ± 141 | 5175 ± 146 | 4249 ± 151 |
| | 10 | 3423 ± 143 | 4106 ± 143 | 4312 ± 155 |
| cpm = Zerfälle pro Minute, Mittelwert aus zwei Bestimmungen ± Standardabweichung<br>nb = nicht bestimmt<br>U = relative biologische Aktivität an IL-2, bezogen auf die vorläufige Internationale Referenz (D.C. Dumonde, B.W. Papermaster, Lymphokin Research 3 (1984), 227) | | | | |

Tabelle 2a

| IL-2 U/Napf | B.B.10 $\mu$g/Napf | Radioaktivität cpm ± SD |
|---|---|---|
| 0 | 0 | 473 ± 237 |
| 1 | 0 | 2788 ± 139 |
| 1 | $2 \times 10^{-4}$ | 2635 ± 136 |
| 1 | $2 \times 10^{-3}$ | 1698 ± 144 |
| 1 | $2 \times 10^{-2}$ | 461 ± 154 |
| 1 | $2 \times 10^{-1}$ | 268 ± 177 |
| 1 | 2 | 312 ± 166 |

Wie hieraus entnommen werden kann, ist der $^3$H-Thymidin-Einbau und damit die DNA-Synthese bei mit B.B.10 behandelten Zellkulturen erheblich niedriger als bei den mit den Kontrollantikörpern behandelten Zellen. Der MAK B.B.10 inhibiert also die PHA-induzierte Proliferation aktivierter humaner T-Blasten.

Beispiel 4b: Inhibierung der IL-2-induzierten Proliferation aktivierter T-Zellen durch B.B.10 und dessen Fab-Fragment

Im folgenden wurde die Wirkungsweise des Fab-Fragments von B.B.10 im Vergleich zum Gesamtmolekül B.B.10 im Hinblick auf die Inhibierung der IL-2-induzierten Proliferation aktivierter T-Zellen wie im vorangegangenen Beispiel getestet, wobei auch der Einfluss von Goat-Antimaus-Serum (GAM) untersucht wurde. Die Ergebnisse sind in nachstehender Tabelle 2b wiedergegeben, wobei wie im vorangegangenen Beispiel der $^3$H-Thymidin-Einbau bei verschiedenen Konzentrationen an Inhibitor ($\mu$g/Napf) bestimmt wurde (cpm), wobei dem Medium jeweils 2 Einheiten IL-2 zugesetzt worden waren.

Tabelle 2b

| Testsubstanz (bei 2U IL-2-Stimulation) | Radioaktivität (cpm±SD) bei den angegebenen Konzentrationen | | | |
|---|---|---|---|---|
| | $10^{-3}$ | $10^{-2}$ | $10^{-1}$ | 1 |
| B.B.10 | 6700 | 4700 | 2300 | 1800 |
| Fab | 7000 | 5100 | 3000 | 1400 |
| Fab + 1 $\mu$g GAM | 6700 | 5300 | 2700 | 1400 |
| Fab + 2 $\mu$g GAM | 7100 | 6700 | 3200 | 1500 |
| GAM 1 $\mu$g | 7000 | | | |
| GAM 2 $\mu$g | 5700 | | | |
| Negative Kontrolle: 20 - Positive Kontrolle: 6900 | | | | |

Wie hieraus entnommen werden kann, erfolgt eine Inhibierung sowohl mit B.B.10 als auch mit dessen Fab-Fragment gleich gut, d.h. der MAK B.B.10 bindet monovalent an den IL-2R. Sein Fab-Fragment kann somit ebenfalls zu klinischen Therapien herangezogen werden, zumal aufgrund seiner kleineren Grösse ein besseres Diffusionsverhalten vorliegt und dadurch eine möglicherweise noch grössere Effektivität bedingt sein kann.

Beispiel 4c: Inhibierung der IL-2-induzierten Proliferation aktivierter T-Zellen durch B.B.10 4 Stunden nach Zugabe von IL-2

$5 \cdot 10^4$ PHA/T-Zellen/Napf von 3 verschiedenen Spendern (Spender 1, 2, 3) wurden zunächst mit 2U IL-2/Napf bei 37°C 4 Stunden inkubiert. Anschliessend wurden die in Tabelle 2c angegebenen Mengen B.B.10 hinzugefügt, ohne dass die Zellen gewaschen worden waren. 18 Stunden später wurde, wie in den vorangegangenen Beispielen der $^3$H-Thymidin-Einbau bestimmt (cpm-Werte). Die Ergebnisse sind in nachfolgender Tabelle 2c angegeben.

## Tabelle 2c

| Konzentration IL-2/B.B.10 | Radioaktivität (cpm ± SD) Spender 1 | Spender 2 | Spender 3 |
|---|---|---|---|
| ohne IL-2 | 667 | 720 | 603 |
| 2U IL2+B.B.10:0 | 8600 | 3300 | 5800 |
| 2U IL2+B.B.10:$10^{-4}$ | 9500 | 3000 | 5200 |
| " +B.B.10:$10^{-3}$ | 8200 | 3000 | 5300 |
| " +B.B.10:$10^{-2}$ | 3600 | 2000 | 3500 |
| " +B.B.10:$10^{-1}$ | 2100 | 1200 | 1600 |
| " +B.B.10:1 | 1600 | 960 | 1200 |
| " +B.B.10:0 | 11000 | 3600 | 5300 |
| Durchschnitt 2U IL2+B.B.10:0 | 9800 | 3400 | 5500 |

Wie hieraus entnommen werden kann, inhibiert B.B.10 auch bei niedrigen Konzentrationen. Das bedeutet, dass selbst nach der Aktivierung der Zellen der B.B.10 die Proliferation inhibiert, da die Internalisierung von IL-2 bei 37°C innerhalb 2 bis 3 Minuten erfolgt.

Beispiel 5a: Inhibierung der gemischten Lymphozyten-Reaktion

$10^5$/ml periphere Blutlymphozyten (PBL) wurden mit $10^5$/ml strahlenbehandeltem (3500 rad) allogenen PBL 5 Tage kokultiviert. Als Kontrolle diente eine syngene Lymphozyten-Reaktion. Geeignete MAK-Konzentrationen wurden an den Tagen 0 bzw. 4 zugesetzt. Die Markierung der Kulturen mit $^3$H-Thymidin erfolgte 18 Stunden vor der Ernte. Der Einbau in die DNA wurde wie in Beispiel 4 gemessen.

Die zugesetzten Mengen B.B.10 sowie die dazugehörigen Radioaktivitätswerte sind in Tabelle 3a aufgezeigt.

Tabelle 3a

| Kultur | MAK B.B.10 μg/Napf | Radioaktivität cpm ± SD B.B.10 zugesetzt am Tag | |
|---|---|---|---|
| | | 0 | 4 |
| Allogene Kultur | 0 | 349 ± 165 | 912 ± 152 |
| gemischte Lymphozyten-Kultur | 0 | 5544 ± 136 | 5616 ± 146 |
| | $2x10^{-2}$ | 1110 ± 136 | 2296 ± 142 |
| | $2x10^{-1}$ | 619 ± 154 | 1827 ± 139 |
| | 2 | 1054 ± 144 | 1555 ± 145 |

Hieraus ist ersichtlich, dass der MAK B.B.10 schon bei niedrigen Konzentrationen wirksam die gemischte Lymphozyten-Reaktion inhibiert.

Beispiel 5b: Vergleich zwischen der Wirksamkeit von B.B.10 und den bekannten anti-Tac-Analogen B-F2, 33B3-1, TÜ 69 und TB 30 hinsichtlich der Inhibierung der gemischten Lymphozyten-Reaktion

Auf die gleiche Art und Weise wie im vorangegangenen Beispiel 5a wurde die Wirksamkeit der nachstehenden MAK's an aktivierten T-Zellen am Tag 0 bzw. am Tag 4 untersucht. Die zum Vergleich herangezogenen MAK's sind anti-Tac-Analoge:

B-F2: CRTS, Besancon (FR)

33B3-1: Inserum U 119, Marseille (FR)

TÜ 69: Biotest, Dreieich (DE)

TB30: CLB, Amsterdam (NL)

Die Ergebnisse sind in nachfolgender Tabelle 3b aufgezeigt:

## Tabelle 3b

### Tag 0

| Antikörper | Konzentration/Napf | |
|---|---|---|
| | 0,02 µg | 0,2 ug |
| | Radioaktivität (cpm ± SD) | |
| B.B.10 | 1000 | 1100 |
| B-F2 | 1300 | 1100 |
| 33B3.1 | 1800 | 2000 |
| TÜ69 | 1200 | 1500 |
| TB30 | 1800 | 1800 |

Negative Kontrolle: 398 – Positive Kontrolle: 5280

### Tag 4

| Antikörper | Konzentration/Napf | |
|---|---|---|
| | 0,02 µg | 0,2 ug |
| | Radioaktivität (cpm ± SD) | |
| B.B.10 | 2300 | 1800 |
| B-F2 | 4600 | 3600 |
| 33B3.1 | 6100 | 4900 |
| TÜ69 | 5400 | 3300 |
| TB30 | 5700 | 4400 |

Negative Kontrolle: 598 – Positive Kontrolle: 6160

Diese Ergebnisse verdeutlichen, das die bekannten Anti-Tac-Analogen B-F2, 33B3.1, TÜ 69 und TB30 bei solch niedrigen Konzentratonen die Proliferation nicht wie B.B.10 inhibieren, wenn der MAK dem Medium erst am Tag 4 zugegeben wird.

Beispiel 6: Scatchard-Analyse: Ermittlung der Affinitätskonstanten

30 µg gereinigter MAK wurde mit $Na^{125}J$ (1 m Ci) in 170 µl PBS inkubiert. Anschliessend wurden 0,4 mg/ml Chloramin T zugesetzt und die Reaktion nach 1 Minute durch Zugabe von 10 µl Natriumbisulfit (0,5

$\mu$g/$\mu$l) abgestoppt.

Der markierte MAK wurde chromatographisch (Sephadex G-25) vom freien Jod abgetrennt. Seine spezifische Radioaktivität betrug 25000 dpm/ng Protein.

Mit verschiedenen Konzentrationen an [125]J-markiertem B.B.10 wurden anschliessend 2,5 x 10[6] periphere PHA-aktivierte humane T-Zellen in 5 ml RPMI 1640/5% Rinderserumalbumin inkubiert.

Um unspezifische Bindungen zu verhindern, wurde unmarkierter B.B.10 MAK in 500-fachem Überschuss zugesetzt. Nach 3 Stunden Inkubationszeit bei 4°C wurden die Zellen dreimal mit RPMI 1640 1% Rinderserumalbumin gewaschen und die gebundene Radioaktivität mit Hilfe eines LKB Wallac (1260 Multigamma Counter) gemessen.

Mit Hilfe der hierbei erhaltenen Werte wurde die Affinitätskonstante ermittelt.

| | PHA-aktivierte Zellen |
|---|---|
| Affinitätskonstante, Kd | $1 \times 10^{-9}$ M |

Beispiel 7: Kompetition von jodiertem B.B.10 mit IL-2 an aktivierten T-Zellen

PHA-aktivierte humane T-Zellen (2,5 x 10[6]) wurden in 1 ml RPMI 1640, 1% Rinderserumalbumin für 3 Stunden bei 4°C inkubiert und IL-2 zugesetzt.

Als Kontrollgruppe dienten obengenannte Kulturen, welchen kein IL-2 zugesetzt wurde.

Anschliessend wurde [125]J-markierter B.B.10 hinzugegeben. Nach 1 Stunde Inkubation wurden die Zellen gewaschen und die gebundene Radioaktivität wie in Beispiel 6 gemessen.

Die Ergebnisse sind in Tabelle 4 zusammengefasst:

Tabelle 4

| [125]J-markierter B.B.10 $\mu$g/ml | B.B.10 $\mu$g/ml | Humanes IL-2 U/ml | Radioaktivität in cpm ± SD |
|---|---|---|---|
| 0,25 | 125 | 0 | 2395 ± 61 |
| 0,25 | 0 | 0 | 30540 ± 3002 |
| 0,25 | 0 | 500 | 28906 ± 3452 |
| 0,25 | 0 | 2500 | 23976 ± 165 |
| 0,25 | 0 | 5000 | 18477 ± 384 |
| 0,25 | 0 | 10000 | 13047 ± 316 |

Hieraus wird ersichtlich, dass sehr hohe Mengen an IL-2 erforderlich sind, um den MAK zu verdrängen (abnehmende Radioaktivitätswerte), was die hohe Spezifität von B.B.10 gegen IL-2 R bestätigt.

III. Erste klinische Versuche mit dem MAK B.B.10

Die Behandlung der Graft versus Host Krankheit (GvH) und der Transplantatabstossung (HvG) sind die Hauptprobleme bei der histokompatiblen und histoinkompatiblen Knochenmarkstransplantation.

Neuere Methoden, die GvH durch polyklonale tierische Antilymphozyten-Seren, durch Depletierung der Spender-T-Zellen aus dem Knochenmark, durch Chemotherapie oder durch anti-T MAK's zu verhindern, haben in vielen Fällen zu starken Nebenwirkungen und zu einer Abstossung des Transplantates geführt.

Um die Immunantwort gegen das Transplantat in vivo zu beeinflussen, können monoklonale Antikörper benutzt werden. Die Anwendung von MAK B.B.10 zur Behandlung von GvH und Transplantatabstossungsreaktionen erwies sich hierbei überraschenderweise als besonders geeignet.

Dadurch ist es möglich, bei Patienten mit diesem Krankheitsbild eine Zusatztherapie zur Unterdrückung der zellulären Immunantwort, z.B. mit Immunsuppressiva wie Antilymphozytenseren, Cyclosporin A oder Chemotherapeutika, z.B. Kortikosteroide, Azathioprin, Methotrexat zu reduzieren oder gänzlich zu vermeiden, welche oft zu schwerwiegenden Nebenwirkungen, wie z.B. einer erhöhten Infektanfälligkeit, führen kann.

Bei den nachstehend beschriebenen klinischen Notfallindikationen wurde den Patienten je 0,1-20 mg MAK B.B.10/Tag, vorzugsweise 2,5-5 mg/Tag intravenös als Infusion über 30 Minuten verabreicht. Die Dosis wurde so gewählt, dass im Patienten Plasmaspiegel von 0,5-5 $\mu$g/ml erreicht wurden.

Die Behandlungsdauer erstreckte sich über 3-30 Tage, bevorzugt jedoch über 7-10 Tage, bis die IL-2-abhängigen Erkrankungserscheinungen zurückgegangen waren. Eine weitere Therapie über diesen Zeitpunkt hinaus erwies sich überraschenderweise als nicht notwendig.

Beispiel 8

Einer Gruppe von 4 Patienten im Alter von 4, 11, 23 und 40 Jahren, von denen 3 an GvH erkrankt waren, wurde die in Tabelle 5 angegebene Menge MAK B.B.10 (verdünnt in Humanalbumin) zum angegebenen Zeitpunkt verabreicht.

Patient 1, 11 Jahre, hatte vor Beginn der Behandlung GvH III.Grades mit gastrointestinaler Dysfunktion. Die Patienten 2 und 3 litten zu Beginn an GvH II.Grades. Patient 4, welchem histoinkompatibles Knochenmark transplantiert worden war und der aufgrund von Nierenschäden nicht mit Cyclosporin A behandelt werden konnte, wurde sofort nach der Transplantation prophylaktisch mit MAK B.B.10 therapiert.

Die verabreichten Mengen und Behandlungsdauer sind in Tabelle 5 aufgezeigt:

Tabelle 5

| Tag | Patient 1 | | Patient 2 | | Patient 3 | | Patient 4 | |
|---|---|---|---|---|---|---|---|---|
| | Tagesdosis MAK in mg | Plasmaspiegel $\mu$g/ml | Tagesdosis MAK in mg | Plasmaspiegel $\mu$g/ml | Tagesdosis MAK in mg | Plasmaspiegel $\mu$g/ml | Tagesdosis MAK in mg | Plasmaspiegel $\mu$g/ml |
| 0 | 7,5 | 0 | 5 | 0 | 5 | 0 | 5 | 0 |
| 1 | 7,5 | 2,9 | 5 | 1,2 | 5 | 0,03 | 5 | 0,07 |
| 2 | 7,5 | 4,7 | 5 | 1,4 | 5 | 0,1 | 5 | 0,14 |
| 3 | 7,5 | 6 | 5 | 2 | 5 | 0,24 | 5 | 0,6 |
| 4 | 7,5 | 7,6 | 5 | 2,2 | 5 | 0,25 | 5 | 0,5 |
| 5 | 7,5 | 8,1 | 5 | 2,3 | 5 | 0,48 | 5 | 0,75 |
| 6 | 7,5 | 8,9 | 5 | 2,9 | 5 | 0,55 | 5 | 1 |
| 7 | 7,5 | 10 | 5 | 3,1 | 5 | 0,96 | 5 | 0,9 |
| 8 | | 3 | 5 | 3,4 | 5 | 1 | 5 | 1,2 |
| 9 | | 2,5 | 5 | 4,1 | 5 | 1,05 | 5 | 1,9 |
| 10 | | 1 | | nb | | 1,1 | | 2,2 |
| 11 | | 0 | | 3,6 | | 0,5 | | |
| 12 | | 0 | | 3,1 | | 0,15 | | |
| 13 | | 0 | | 1,3 | | 0 | | |
| 14 | | 0 | | 0,6 | | 0 | | |
| 15 | 5 | 0 | | 0,1 | | | | |
| 16 | | 1,5 | | | | | | |
| 17 | 5 | 0 | | | | | | |
| 18 | | nb | | | | | | |
| 19 | 5 | 0 | | | | | | |
| 20 | | 0,15 | | | | | | |
| 21 | 5 | 0 | | | | | | |
| 22 | | 0,3 | | | | | | |
| 23 | 5 | 0 | | | | | | |
| 24 | | 0,3 | | | | | | |
| nb = nicht bestimmt | | | | | | | | |

Ergebnis:

Überraschenderweise bildeten sich bei den Patienten 1-3 die akuten Symptome der GvH innerhalb der ersten 3 Tage nach Beginn der MAK-Behandlung zurück. Patient 1 wies bereits nach 24 Tagen keine Symptome mehr auf. Bei Patient 2 konnte die Behandlung nach 15 Tagen, bei Patient 3 nach 14 Tagen bei völliger Symptomfreiheit abgeschlossen werden. Patient 4, welcher prophylaktisch behandelt wurde, entwikkelte keine HvG nach Abschluss der Behandlung (10 Tage).

Ebenfalls nicht zu erwarten war, dass bei keinem der Patienten eine therapiebeeinträchtigende Nebenwirkung aufgetreten ist.

Beispiel 9

Eine Gruppe von 3 Patienten (5 bis 7) im Alter von 5, 7 und 12 Jahren, wurde zur Behandlung der Transplantatabstossung nach Knochenmarkstransplantationen mit MAK B.B.10 therapiert.
Bei zwei dieser Patienten war eine histokompatible Knochenmarkstransplantation, bei dem dritten eine haploide Knochenmarkstransplantation vorgenommen worden. Aufgrund des Alters wurde bei diesen Patienten eine Dosierung des MAK B.B.10 von nur 2,5 mg/Tag eingesetzt.
Die verabreichten Mengen und die Dauer der Behandlung sind in Tabelle 6 zusammengefasst:

Tabelle 6

| Tag | Patient 5 | | Patent 6 | | Patent 7 | |
|---|---|---|---|---|---|---|
| | Tagesdosis MAK in mg | Plasmaspiegel $\mu$g/ml | Tagesdosis MAK in mg | Plasmaspiegel $\mu$g/ml | Tagesdosis MAK in mg | Plasmaspiegel $\mu$g/ml |
| 0 | 2,5 | 0 | 2,5 | 0 | 2,5 | 0 |
| 1 | 2,5 | 1,7 | 2,5 | 1 | 2,5 | 1,2 |
| 2 | 2,5 | 1,9 | 2,5 | 1,4 | 2,5 | 1,8 |
| 3 | 2,5 | 2,7 | 2,5 | 1,9 | 2,5 | 2,7 |
| 4 | 2,5 | 2,5 | 2,5 | 3,6 | 2,5 | 4,1 |
| 5 | 2,5 | 4,5 | 2,5 | 2,3 | 2,5 | nb |
| 6 | | nb | 2,5 | 3,8 | 2,5 | nb |
| 7 | 2,5 | 1,5 | 2,5 | nb | 2,5 | 3 |
| 8 | | 0,9 | 2,5 | 4,4 | | 1,3 |
| 9 | 2,5 | 1,1 | | 3,9 | | 2 |
| 10 | | 1,6 | | 3,7 | | |
| 11 | | 0,7 | | 2,6 | | |
| 12 | | | | 0,9 | | |
| nb = nicht bestimmt | | | | | | |

Ergebnis:

Bei Patient 5, bei welchem eine haploide Knochenmarkstransplantation vorgenommen worden war, wurde die Abstossungsreaktion rasch unterdrückt und das Transplantat angenommen.
Patient 6 zeigte keine Abstossungsreaktion. Das Transplantat wurde vom Immunsystem toleriert.
Bei Patient 7, der eine autologe Knochenmarkstransplantation erhalten hatte, war kein Anwachsen des Transplantats zu sehen.
Diese Ergebnisse der ersten Verwendung in klinischen Notfällen sowie der Tests bezüglich der Affinität und der biologischen Aktivität des MAK B.B.10 zeigen, dass der erfindungsgemässe MAK sowohl als Prophylaktikum als auch als Therapeutikum bei der Transplantatabstossung und der GvH und/oder HvG wirksam eingesetzt werden kann und allen anderen bisher bekannten Therapiemethoden klar überlegen ist.
Im Gegensatz zu bisher beschriebenen Therapien mit MAK's treten hierbei keine Nebenwirkungen auf. Der MAK kann dabei in Konzentrationen eingesetzt werden, die niedriger sind als bisher eingesetzte Mengen (s. J.P.Soulillou in The Lancet, 13. Juni, 1339 (1987). Der therapeutische Index ist also entsprechend höher. Bisher konnte aufgrund der Notfallindikation keine Dosisabhängigkeit der klinischen Effizienz bestimmt werden; möglicherweise ist die effektive Dosis noch geringer als bisher gefunden.
Da die Patienten insgesamt mit weniger Fremdprotein belastet wurden, ist die Wahrscheinlichkeit der Bildung von Antikörpern gegen den IL-2 R-Antikörper erheblich geringer ohne die Effizienz des Mittels zu beeinträchtigen.
Eine mit schweren Nebenwirkungen belastete Zusatztherapie mit anderen Pharmaka erwies sich als nicht notwendig.

Beispiel 10

Der MAK B.B.10 wurde ferner auf seine Wirksamkeit bei 26 Patienten untersucht, welche nach einer Knochenmarkstransplantation an akuter GvH grösser II. Grades litten und refraktär gegenüber der Behandlung mit Corticoiden waren. Den Patienten wurden über einen Zeitraum von 10 Tagen je 5 mg B.B.10/Tag verabreicht. Während dieser Behandlung wurden keine Nebenwirkungen beobachtet.

Die Ergebnisse dieser Studie sind nachfolgend zusammengefasst:

Positive Antwort:        18 von 26 (69,2%), 12 davon mit GvH II. Grades, 6 mit GvH III. Grades

Partielle Antwort:       5 von 26 (19,2%), 1 mit GvH II. Grades, 2 mit GvH III. Grades, 2 mit GvH IV. Grades

Non-Responder:         3 von 26 (11,5%), 2 mit GvH III. Grades, 1 mit GvH IV. Grades

Dabei wurde festgestellt, dass der Zeitraum zwischen dem Beginn der GvH-Anzeichen und dem Beginn der Behandlung äusserst wichtig ist: Für positive Reaktionen betrug dieser im Durchschnitt 13,7 Tage, während im Fall der negativen Antwort im Durchschnitt 70 Tage dazwischen lagen.

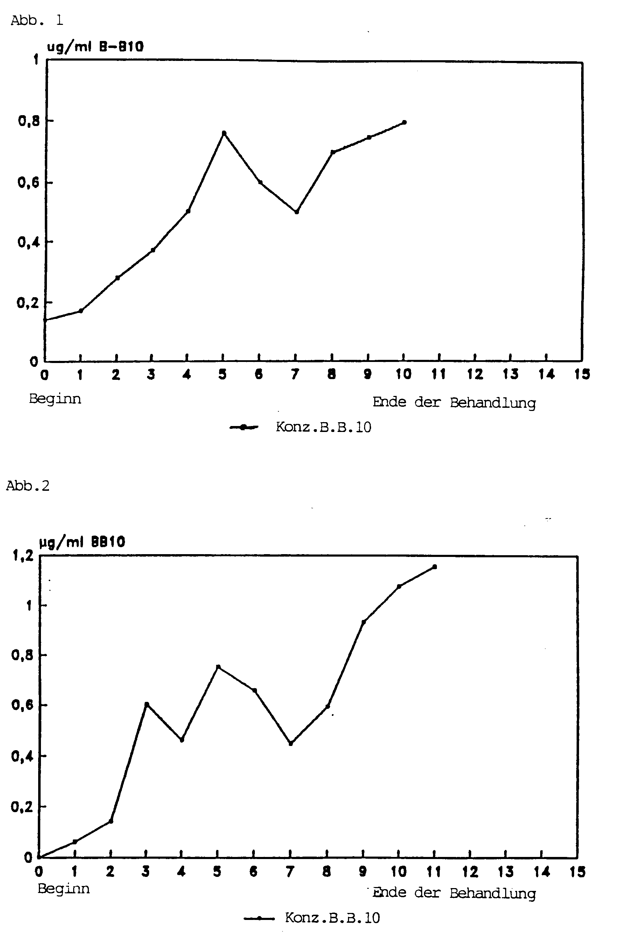

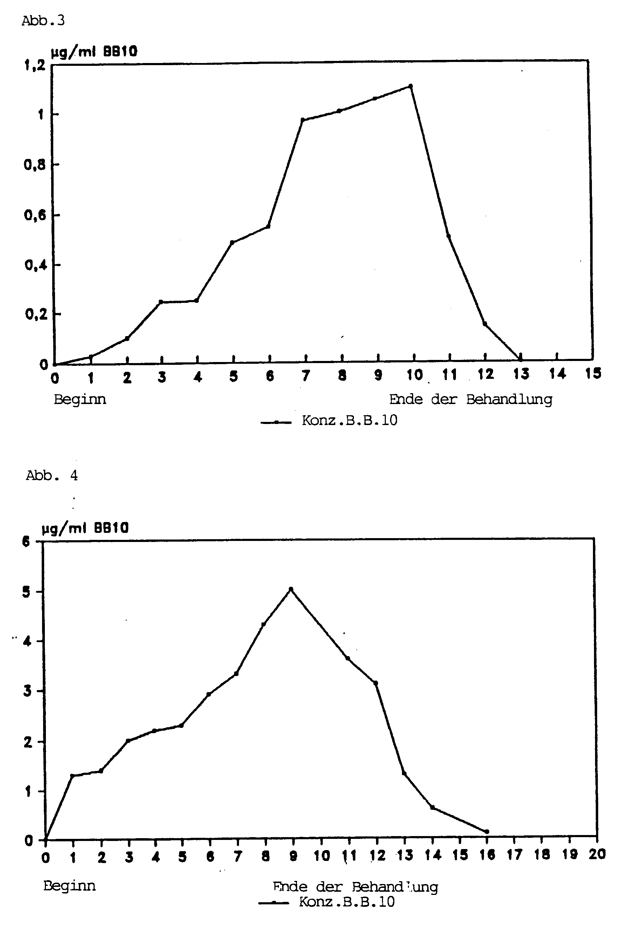

In den nachfolgenden Abbildungen 1 bis 4 ist der Gehalt an B.B.10 (Serumspiegel) von 4 Patienten in Abhängigkeit von der Behandlungsdauer aufgezeigt. Wie hieraus ersichtlich ist, bleibt über die gesamte Zeit der B.B.10-Spiegel aufrechterhalten (B.B.10-Zirkulation), was für andere bekannte MAK's nicht der Fall ist.

**Patentansprüche**

**Patentansprüche für folgende Vertragsstaaten : AT, BE, CH, LI, DE, FR, GB, GR, IT, LU, NL, SE**

**1.** Hybridoma-Zellinie mit der C.N.C.M.-Hinterlegungsnummer I-752, dadurch gekennzeichnet, dass sie einen monoklonalen Maus-IgG$_1$-Antikörper herstellt, welcher den humanen IL-2-Rezeptor erkennt und mit der 55 KDa-Untereinheit des IL-2-Rezeptors spezifisch wechselwirkt, die Bindung von humanem IL-2 an seinen Rezeptor inhibiert und/oder verdrängt und eine bereits ablaufende allogene Lymphozyten-Reaktion inhibiert.

**2.** Hybridoma-Zellinie nach Anspruch 1, gekennzeichnet durch daraus isolierbare Varianten, welche die Hauptklassen IgG$_{2a}$, IgG$_{2b}$, IgG$_3$, IgM und anderer Antikörperklassen herstellt, die die gleichen Eigenschaften besitzen.

**3.** Monoklonaler Antikörper der Klasse IgG$_1$, erhältlich aus der Zellinie gemäss Anspruch 1, gekennzeichnet durch spezifische Wechselwirkung mit humanen Zellen, welche den IL-2-Rezeptor exprimieren.

**4.** Chimärer monoklonaler Antikörper, dadurch gekennzeichnet, dass der konstante Teil aus humanem Ig und der variable und insbesondere der hypervariable Teil aus dem monoklonalen Maus-IgG$_1$-Antikörper nach Anspruch 3 besteht.

**5.** Verwendung eines monoklonalen Antikörpers nach einem der Ansprüche 3 oder 4 zur Herstellung eines Mittels zur Therapie, Prophylaxe und Diagnose von IL-2-abhängigen Erkrankungen beim Menschen.

**6.** Verwendung gemäß Anspruch 5 zur Behandlung von Transplantatabstossungen und zwar der Host versus Graft- und Graft versus Host-Krankheit, insbesondere Knochenmarksabstossungen beim Menschen.

**Patentansprüche für folgenden Vertragsstaat : ES**

**1.** Verfahren zur Herstellung einer Hybridoma-Zellinie mit der C.N.C.M.-Hinterlegungsnummer I-752, welche einen monoklonalen Maus-IgG$_1$-Antikörper herstellt, der den humanen IL-2-Rezeptor erkennt und mit der 55 KDa-Untereinheit des IL-2-Rezeptors spezifisch wechselwirkt, die Bindung von humanem IL-2 an seinen Rezeptor inhibiert und/oder verdrängt und eine bereits ablaufende allogene Lymphozyten-Reaktion inhibiert, dadurch gekennzeichnet, daß man in an sich bekannter Weise T-Zellen aus humanen peripheren Blutlymphozyten isoliert, inkubiert, die so erhaltenen kultivierten Zellen weiblichen BALB/c-Mäusen verabreicht, die nach Immunisierung erhaltenen Milzzellen entnimmt und anschließend mit Maus-Myeloma-Zellen fusioniert und inkubiert.

**2.** Verfahren nach Anspruch 1, dadurch gekennzeichnet, daß man aus der nach Fusion erhaltenen Zellinie Varianten isoliert, welche die Hauptklassen IgG$_{2a}$, IgG$_{2b}$, IgG$_3$, IgM und anderer Antikörperklassen herstellt, die die gleichen Eigenschaften besitzen.

3.  Verfahren zur Herstellung eines monoklonalen Antikörpers der Klasse $IgG_1$, dadurch gekennzeichnet, daß man aus der Zellinie, hergestellt nach dem Verfahren gemäß Anspruch 1, nach der Zellfusion aus dem Überstand der fusionierten inkubierten Zellsuspension den Antikörper B.B. 10 isoliert und in vivo durch Injektion in BALB/c-Mäuse in großen Mengen in an sich bekannter Weise produziert, wobei dieser Antikörper eine spezifische Wechselwirkung mit humanen Zellen, welche den IL-2-Rezeptor exprimieren, aufweist.

4.  Verfahren zur Herstellung chimärer monoklonaler Antikörper, dadurch gekennzeichnet, daß man einen konstanten Teil aus humanem Ig und einem variablen und insbesondere hypervariablen Teil aus dem monoklonalen Maus-$IgG_1$-Antikörper nach Anspruch 3 kombiniert.

5.  Verwendung eines monoklonalen Antikörpers, hergestellt nach einem der Ansprüche 3 oder 4 zur Herstellung eines Mittels zur Therapie, Prophylaxe und Diagnose von IL-2-abhängigen Erkrankungen beim Menschen.

6.  Verwendung gemäß Anspruch 5 zur Behandlung von Transplantatabstossungen und zwar der Host versus Graft- und Graft versus Host-Krankheit, insbeonsere Knochenmarksabstossungen beim Menschen.

## Claims
## Claims for the following Contracting States : AT, BE, CH, LI, DE, FR, GB, GR, IT, LU, NL, SE

1.  Hybridoma cell line having the C.N.C.M. file number I-752, characterized in that it produces a monoclonal mouse-$IgG_1$ antibody which recognises the human IL-2 receptor and specifically interacts with the 55 KDa sub-unit of the IL-2 receptor, inhibits and/or displaces the binding of human IL-2 to its receptor and inhibits an already proceeding allogenic lymphocytic reaction.

2.  Hybridoma cell line according to claim 1, characterized by variations which can be isolated therefrom, which produce the main classes $IgG_{2a}$, $IgG_{2b}$, $IgG_3$, IgM and other classes of antibodies which have the same properties.

3.  Monoclonal antibody of class $IgG_1$ obtainable from the cell line according to claim 1, characterized by specific interaction with human cells which express the IL-2 receptor.

4.  Chimeric monoclonal antibody, characterized in that the constant part consists of human Ig and the variable and in particular the hypervariable part consists of the monoclonal mouse-$IgG_1$ antibody according to claim 3.

5.  Use of a monoclonal antibody according to one of the claims 3 or 4 for the production of an agent for therapy, prophylaxis and diagnosis of IL-2-dependent diseases in humans.

6.  Use according to claim 5 for the treatment of transplant rejections, namely of the host versus graft and graft versus host disease, in particular bone marrow rejections in humans.

## Claims for the following Contracting State : ES

1.  A process for the production of a hybridoma cell line having the C.N.C.M. file number I-752 which produces a monoclonal mouse $IgG_1$ antibody which recognises the human IL-2 receptor and specifically interacts with the 55 KDa subunit of the IL-2 receptor, inhibits and/or displaces the binding of human IL-2 to its receptor and inhibits an already proceeding allogenic reaction, characterized in that T-cells from human peripheral blood lymphocytes are isolated in known manner and incubated, the cultivated cells thus obtained are administered to female BALB/c mice, and the spleen cells obtained after immunisation are removed and subsequently fused with mouse myeloma cells and incubated.

2.  A process according to claim 1, characterized in that variations which produce the main classes $IgG_{2a}$, $IgG_{2b}$, $IgG_3$, IgM and other classes of antibodies which have the same properties are isolated from the cell line obtained after fusion.

3. A process for the production of a monoclonal antibody of class IgG$_1$, characterized in that from the cell line produced by the process according to claim 1, the antibody B.B. 10 is isolated from the supernatant layer of the fused incubated cell suspension after after cell fusion and produced in large quantities in vivo by injection into BALB/c mice in known manner, this antibody having a specific interaction with human cells which express the IL-2 receptor.

4. A process for the production of chimeric monoclonal antibodies, characterized in that a constant part of human Ig and a variable and in particular hypervariable part of the monoclonal mouse IgG$_1$ antibody claimed in claim 3 are combined.

5. Use of a monoclonal antibody produced according to one of the claims 3 or 4 for the production of an agent for therapy, prophylaxis and diagnosis of IL-2-dependent diseases in humans.

6. Use according to claim 5 for the treatment of transplant rejections, namely of the host versus graft and graft versus host disease, in particular bone marrow rejections in humans.

**Revendications**

**Revendications pour les Etats contractants suivants : AT, BE, CH, LI, DE, FR, GB, GR, IT, LU, NL, SE**

1. Lignée cellulaire d'hyhridome portant le numéro de dépôt C.N.C.M. I-752, caractérisée en ce qu'elle produit un anticorps monoclonal IgG$_1$ de souris qui reconnaît le récepteur à l'IL-2 humain et présente une interaction spécifique avec la sous-unité de 55 KDa du récepteur à l'IL-2, inhibe et/ou défait la fixation de l'IL-2 humaine à son récepteur et inhibe une réaction lymphocytaire allogène déjà en cours.

2. Lignée cellulaire d'hybridome selon la revendication 1, caractérisée par des variantes isolables qui produisent les classes principales IgG$_{2a}$, IgG$_{2b}$, IgG$_3$, IgM et d'autres classes d'anticorps possédant les mêmes propriétés.

3. Anticorps monoclonal de la classe des IgG$_1$ qu'il est possible d'obtenir à partir de la lignée cellulaire selon la revendication 1, caractérisé par une interaction spécifique avec des cellules humaines qui expriment le récepteur à l'IL-2.

4. Anticorps monoclonal chimère, caractérisé en ce que la partie constante est faite d'Ig humaine et la partie variable et notamment la partie hypervariable de l'anticorps monoclonal IgG$_1$ de souris selon la revendication 3.

5. Utilisation d'un anticorps monoclonal selon une des revendications 3 ou 4, pour la préparation d'un agent de traitement, prophylaxie et diagnostic de pathologies IL-2-dépendantes chez l'homme.

6. Utilisation selon la revendication 5 pour le traitement de rejets de greffes, à savoir de la réaction de l'hôte contre le greffon et du greffon contre l'hôte, notamment des rejets de moelle osseuse chez l'homme.

**Revendications pour l'Etat contractant suivant : ES**

1. Procédé de préparation d'une lignée cellulaire d'hybridome portant le numéro de dépôt C.N.C.M. I-752, qui produit un anticorps monoclonal IgG$_1$ de souris qui reconnaît le récepteur à l'IL-2 humain et présente une interaction spécifique avec la sous-unité de 55 KDa du récepteur à l'IL-2, inhibe et/ou défait la fixation de l'IL-2 humaine à son, récepteur et inhibe une réaction lymphocytaire allogène déjà en cours, caractérisé en ce qu'on isole de façon connue en soi des cellules T de lymphocytes sanguins périphériques humains, qu'on les incube, qu'on administre les cellules cultivées obtenues à des souris BALB/c femelles, qu'on prélève les cellules spléniques obtenues après l'immunisation et qu'on les fait ensuite fusionner avec des cellules de myélome de souris et qu'on incube.

2. Procédé selon la revendication 1, caractérisé en ce qu'on isole de la lignée cellulaire obtenue après fusion des variantes qui produisent les classes principaes IgG$_{2a}$, IgG$_{2b}$, IgG$_3$, IgM et d'autres classes d'anticorps possédant les mêmes propriétés.

3. Procédé de préparation d'un anticorps monoclonal de la classe des IgG$_1$, caractérisé en ce qu'on isole l'anticorps B.B. 10 de la lignée cellulaire préparée selon le procédé selon la revendication 1, après la fusion cellulaire, dans le surnageant de la suspension cellulaire incubée et fusionnée et qu'on le produit in vivo par injection à des souris BALB/c en grandes quantités, de façon connue en soi, cet anticorps ayant une interaction spécifique avec des cellules humaines exprimant le récepteur à l'IL-2.

4. Procédé de préparation d'anticorps monoclonaux chimères, caractérisé en ce qu'on associe une partie constante provenant d'Ig humaine et une partie variable et notamment hypervariable provenant de l'anticorps monoclonal IgG$_1$ de souris selon la revendication 3.

5. Utilisation d'un anticorps monoclonal préparé selon l'une des revendications 3 ou 4 pour la préparation d'un agent de traitement, prophylaxie et diagnostic de pathologies IL-2-dépendantes chez l'homme.

6. Utilisation selon la revendication 5 pour le traitement de rejets de greffes, à savoir de la réaction de l'hôte contre le greffon et du greffon contre l'hôte, notamment des rejets de moelle osseuse chez l'homme.

Abb. 1

Konz.B.B.10

Abb.2

Konz.B.B.10

16

Abb.3

Abb. 4